Europäisches Patentamt

European Patent Office

Office européen des brevets

(11) Veröffentlichungsnummer: **0 295 521 B1**

## (12) EUROPÄISCHE PATENTSCHRIFT

(45) Veröffentlichungstag der Patentschrift: **02.09.92**

(21) Anmeldenummer: **88108993.2**

(22) Anmeldetag: **06.06.88**

(51) Int. Cl.⁵: **A61B 8/14**

(54) **Frei beweglicher Ultraschall-Applikator, insbesondere für die Hüftdiagnostik.**

(30) Priorität: **15.06.87 DE 3719919**

(43) Veröffentlichungstag der Anmeldung:
**21.12.88 Patentblatt 88/51**

(45) Bekanntmachung des Hinweises auf die
Patenterteilung:
**02.09.92 Patentblatt 92/36**

(84) Benannte Vertragsstaaten:
**AT CH DE FR GB LI NL**

(56) Entgegenhaltungen:
**DE-A- 3 227 624**

**IEEE SPECTRUM, Band 8, August 1974, Seiten 62-66; R.R. JURGEN: "Ultrasonics in medicine: strong; getting stronger"**

(73) Patentinhaber: **Graf, Reinhard, Prof. Dr.
Landessonderkrankenhaus
A-8852 Stolzalpe(AT)**

(72) Erfinder: **Graf, Reinhard, Dr.
Landessonderkrankenhaus
A-8852 Stolzalpe(AT)**
Erfinder: **Soldner, Richard
Richard Wagner-Strasse 7
W-8522 Herzogenaurach(DE)**

(74) Vertreter: **Fuchs, Franz-Josef, Dr.-Ing. et al
Postfach 22 13 17
W-8000 München 22(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf einen an ein Echosichtbild-Aufzeichnungsgerät anschließbaren Ultraschall-Applikator, insbesondere für die Hüftdiagnose an einem Patienten, mit einem Ultraschall-Übertrager zur zeilenweisen Ultraschall-Abtastung, der in einer Halterung gehaltert und durch einen Motorantrieb um eine in der Abtastebene gelegene Drehachse drehbar ist.

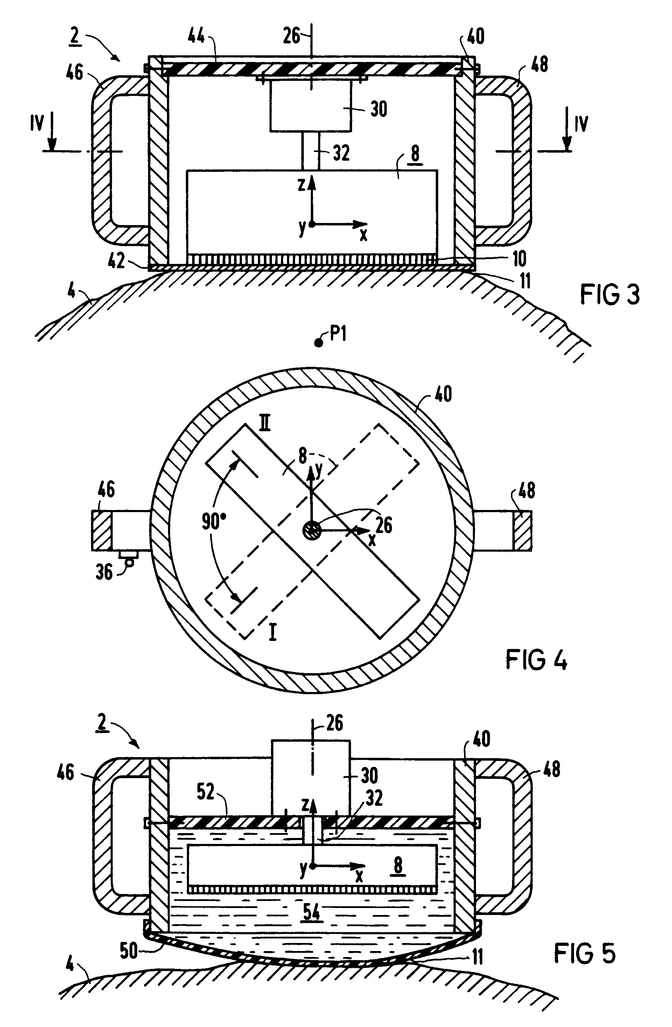

Ein solcher Ultraschall-Applikator ist aus der deutschen Offenlegungsschrift 30 12 173, insbesondere Fig. 3, bekannt. Dort ist ein Sektorabtastkopf vorgesehen, der durch einen Schwenkmotor in einem vorgegebenen Winkelbereich um eine Schwenkachse hin- und hergeschwenkt wird. Dabei erfolgt eine zeilenweise Ultraschall-Abtastung. Der genannten Schwenkbewegung ist eine Drehbewegung des Abtastkopfes um eine zentrale Drehachse überlagert, welche durch einen Drehmotor herbeigeführt wird. Auf diese Weise wird der Ultraschall-Abtastsektor gedreht, und es ergibt sich ein Abtastkegel. Dieser Ultraschall-Applikator findet Anwendung im Brustbereich eines Patienten zur Gewinnung subcostaler Schnittbilder, z.B. des Herzens oder der Leber. Er ist dabei an ein Echosichtbild-Auswerte- und -Aufzeichnungsgerät angeschlossen.

Ein Ultraschall-Applikator mit einem um eine Achse rotierbaren linearen Ultraschallwandler-Array, das in einem topfförmigen Gehäuse untergebracht ist, ist aus der deutschen Offenlegungsschrift 32 27 624 bekannt. Das mit einer Ultraschall-Koppelflüssigkeit gefüllte Gehäuse ist endseitig von einer flexiblen Membran verschlossen. Dieser Applikator dient zur Untersuchung der weiblichen Brust.

Auf dem Arbeitsgebiet der Hüftdiagnose mittels Ultraschall (vgl. R. Graf: "Sonographic Diagnosis of Hip-Dysplasia and Hip-Dislocation", Publikation der Siemens AG, Unternehmensbereich Medizinische Technik, D-8520 Erlangen, Bestell-Nr. A 19100-M 2400-B454-01-7600, S. 1-29, 1986) wird mit einem in der Nähe des Hüftknochens des Patienten aufgelegten Ultraschall-Applikator und nachgeschaltetem Echosichtbildgerät ein Echosichtbild erzeugt, das dem Arzt Aufschluß über Anomalien und Erkrankungen des Hüftgelenks des Patienten gibt. Es hat sich bei Ultraschall-Untersuchungen an Patienten im Säuglingsalter gezeigt, daß die relative Lage und/oder der Abstand von drei charakteristischen Punkten im Hüftgelenk Aussagen darüber zulassen, ob das Auftreten einer Hüftluxation zu befürchten ist. Bei diesen Untersuchungen wird zunächst durch geeignetes Auflegen des Applikators der erste charakteristische Punkt ins Ultraschallbild gebracht. Der Arzt dreht nun den Applikator um eine Achse, die in der Abtast- oder Schnittebene liegt und die durch den ersten charakteristischen Punkt verläuft. Unter Beobachtung des Sichtbildes dreht der Arzt den Applikator so lange, bis die beiden anderen charakteristischen Punkte als Echopunkte auf dem Bildschirm auftauchen. Jetzt ist die richtige Schnittebene, in der alle drei Punkte liegen, eingestellt. Das entsprechende Echosichtbild wird nun automatisch aufgezeichnet und vom Arzt ausgewertet.

Das Einstellen der richtigen Schnittebene mit den drei charakteristischen Punkten wurde bisher vom Arzt freihändig vorgenommen (vgl. Fig. 24 bis 27 der genannten Siemens-Publikation). Es erfordert neben viel Erfahrung eine ruhige Hand des Arztes, aber auch ein ruhiges Verhalten des Patienten. Gerade letzteres ist bei Patienten im Säuglingsalter kaum gewährleistet. Wünschenswert wäre hier ein Ultraschall-Applikator, der die eindeutige und reproduzierbare Ultraschall-Bilddarstellung der Hüfte des Babys, insbesondere bei Vorsorgeuntersuchungen hinsichtlich einer Hüftluxation, ermöglicht. Darüber hinaus sollte bei der Untersuchung eine Anpassung der Lage des Ultraschall-Applikators an die Lage des (potentiell unruhigen) Patienten möglich sein. Insgesamt gesehen sollte der Applikator die Hüftsonographie erleichtern.

Der Erfindung liegt die Aufgabe zugrunde, den eingangs genannten Ultraschall-Applikator so weiterzubilden, daß er allseits frei beweglich ist und auch bei einem unruhigen Patienten die Aufzeichnung zumindest eines Schnittbildes in definierter Stellung erlaubt. Insbesondere soll der Ultraschall-Applikator so weitergebildet werden, daß er zur Darstellung der erwähnten charakteristischen Punkte bei der Untersuchung der Hüfte eines Säuglings geeignet ist. Der Applikator soll bevorzugt für Routine-Untersuchungen eingesetzt werden können. Er soll daher das Auffinden einer interessierenden Schnittebene im Patienten erleichtern.

Diese Aufgabe wird erfindungsgemäß dadurch gelöst, daß die Halterung frei bewegbar und mit zwei in einer Ebene liegenden Handgriffen versehen ist, daß der Ultraschall-Übertrager zwischen den Handgriffen angeordnet und dabei um die Drehachse, die parallel zur Ebene der Handgriffe liegt, schwenkbar ist, und daß der Motorantrieb zum Schwenken des Ultraschall-Übertragers um die Drehachse relativ zur Halterung vorgesehen ist.

Mit einem solchen Ultraschall-Applikator ist ein leichtes und schnelles Arbeiten bei der Aufnahme von Ultraschall-Schnittbildern in gewünschten Schnittebenen möglich. Diese Arbeiten können schon nach kurzer Einarbeitung, ggf. von einem Assistenten, durchgeführt werden. Der Ultraschall-Applikator eignet sich insbesondere für die Hüftdiagnose bei unruhigen Patienten wie speziell Säuglingen, da der Arzt oder Assistent mit beiden Händen den Applikator führen und seine Aufmerksamkeit voll dem Sichtbild widmen kann.

Nach einer ersten grundlegenden Weiterbildung ist vorgesehen, daß die Halterung als U-förmiger, frei bewegbarer Haltebügel ausgebildet ist, daß die Schenkel des Haltebügels als die Handgriffe ausgebildet sind, und daß der Ultraschall-Übertrager zwischen den Schenkeln des Haltebügels angeordnet sind und dabei um die Drehachse, die parallel zu den Schenkeln liegt, schwenkbar ist. Nach einer weiteren grundlegenden Weiterbildung ist dagegen vorgesehen, daß die Halterung einen starren ringförmigen Grundkörper aufweist, an dessen Außenseite die Handgriffe angebracht sind.

Bevorzugt wird so vorgegangen, daß an zumindest einem Handgriff, z.B. an einem Schenkel des Haltebügels oder an einem henkelförmigen Griff des Grundkörpers, ein Auslöser oder Schalter zum Auslösen der Schwenkbewegung und/oder für die Bildaufzeichnung angeordnet ist. Prinzipiell könnte auch ein Fußschalter oder -auslöser verwendet werden; doch der genannte, von Hand zu betätigende Auslöser oder Schalter ermöglicht eine größere Freizügigkeit beim Arbeiten.

Von Bedeutung ist es, daß ein übliches (konventionelles) Ultraschallgerät mit den erfindungsgemäßen Mitteln nachgerüstet werden kann. Der Ultraschall-Applikator gemäß der Erfindung kann somit insbesondere als Zusatzgerät ausgeführt werden, so daß ein kostspieliges Sondergerät entbehrlich ist.

Das bevorzugte Verfahren zum Betrieb des Applikators zeichnet sich dadurch aus, daß

a) die Applikationsfläche auf das zu untersuchende Objekt aufgesetzt wird, wobei der Ultraschall-Übertrager gegenüber der Halterung arretiert ist,

b) der Applikator auf dem Objekt verschoben wird, bis ein interessierender Punkt auf dem Sichtbild erscheint,

c) das Schwenken des Ultraschall-Übertragers um die Drehachse ausgelöst wird,

d) beim Schwenken die Handgriffe festgehalten sowie gleichzeitig Schnittbild-Aufnahmen gemacht und aufgezeichnet werden, und

e) die Schnittbild-Aufnahmen nacheinander aus dem Bildspeicher abgerufen werden.

Weitere vorteilhafte Ausgestaltungen der Erfindung sind in den Unteransprüchen niedergelegt.

Ausführungsbeispiele der Erfindung werden im folgenden anhand von fünf Figuren näher erläutert. Gleiche und gleichwirkende Bauteile sind mit denselben Bezugszeichen versehen. Es zeigen:

Fig. 1 einen Ultraschall-Applikator nach der ersten grundlegenden Weiterbildung für die Hüftdiagnose an einem Patienten in einer Seitenansicht;

Fig. 2 den Ultraschall-Applikator nach Fig. 1 in Aufsicht;

Fig. 3 einen Ultraschall-Applikator nach der zweiten grundlegenden Weiterbildung für die Hüftdiagnose in einer seitlichen Schnittdarstellung;

Fig. 4 den Ultraschall-Applikator nach Fig. 3 in einem dazu senkrechten Schnitt; und

Fig. 5 einen weiteren Ultraschall-Applikator nach der zweiten grundlegenden Weiterbildung für die Hüftdiagnose ebenfalls in seitlicher Schnittdarstellung.

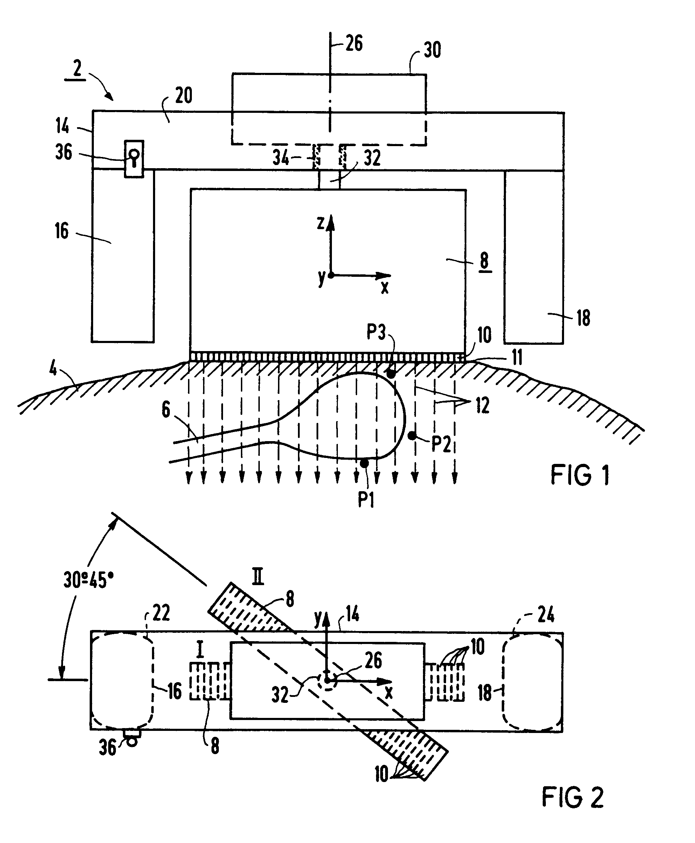

In den Figuren 1 und 2 ist ein Ultraschall-Applikator 2 gezeigt, der insbesondere für die Hüftdiagnose an einem Patienten 4 vorgesehen ist. Mit ihm läßt sich weitgehend automatisch, sehr rasch und reproduzierbar arbeiten. Der Hüftknochen des Patienten 4 ist mit 6 bezeichnet, und die aufzufindenden und im Schnittbild darzustellenden charakteristischen Punkte sind mit den Zeichen P1, P2, P3 belegt. Vorliegend ist angenommen, daß sich diese Punkte P1, P2, P3 in der Papierebene von Fig. 1 befinden; weiter ist angenommen, daß auch die Abtast- oder Schnittebene x, z des Ultraschall-Applikators 2 in Fig. 1 bereits mit der Papierebene zusammenfällt.

Kernstück des Ultraschall-Applikators 2 ist ein Ultraschall-Übertrager oder -Wandler 8 zur zeilenweisen Ultraschall-Abtastung des Patienten 4. Der Ultraschall-Übertrager 8 ist hier speziell ein lineares Array. Dessen Wandlerelemente 10 dienen hier als Applikationsfläche 11, die über ein Ankoppelgel an den Patienten 4 angekoppelt sind. Sie ermöglichen in bekannter Weise eine Parallelabtastung in Abtastrichtung x. Die y-Richtung steht senkrecht auf der Papierebene in Fig. 1. Die einzelnen parallelen Abtastzeilen für Senden und Empfang sind in Fig. 1 durch gestrichelte Pfeile 12 verdeutlicht. Der Ultraschall-Applikator 2 ist an ein (nicht gezeigtes) Echosichtbild-Aufzeichnungsgerät mit Bildspeicher angeschlossen. Statt eines linearen Arrays kann auch ein Sektor-Scanner eingesetzt werden.

Der Ultraschall-Übertrager 8 ist in einer Halterung untergebracht und gehalten. Diese Halterung ist hier speziell als U-förmiger, frei beweglicher Haltebügel 14 ausgebildet. Dieser Haltebügel 14 besitzt zwei parallele Schenkel 16 und 18 sowie einen Verbindungssteg 20. Die beiden Schenkel 16, 18 des Haltebügels 14 sind als Handgriffe ausgebildet. Das kann z.B. dadurch geschehen, daß die Schenkel 16, 18 gerundet sind, was in Fig. 2 durch die Rundungen 22 und 24 angedeutet ist. Das kann auch durch Griffmulden (nicht gezeigt) oder eine rauhe Oberflächenbeschichtung geschehen. Auf jeden Fall ist es so, daß der linke Schenkel 16 von der linken Hand und der rechte Schenkel 18 von der rechten Hand des Bedienenden (Arzt, Assistent, Krankenschwester) einfach umfaßt werden kann. Der Haltebügel 14 ist ein in sich starres Gebilde und kann aus Metall bestehen.

Der Ultraschall-Übertrager 8 ist etwa in der Mitte zwischen den Schenkeln 16, 18 des Haltebügels 14 angeordnet und dort um eine in der Abtastebene x, z gelegene Drehachse 26 dreh- oder schwenkbar. Die Drehachse 26 liegt also parallel zu den beiden Schenkeln 16, 18 und damit parallel zur z-Achse in Fig. 1 und 2.

Ein Motorantrieb 30 ist vorgesehen, um den zunächst arretierten Ultraschall-Übertrager 8, der als Linear- oder Sektor-Scanner ausgeführt sein kann, zwecks Drehung der Schnittebene x, z um die Drehachse 26 zu drehen oder zu schwenken. Die Drehung oder das Schwenken erfolgt relativ zum Haltebügel 14, der dabei von den Händen des Bedienenden festgehalten wird. Zum Schwenken um die Drehachse 26 dient eine Welle 32, die den Motorantrieb 30 mit dem Ultraschall-Wandler 8 verbindet. Diese Antriebswelle 32 ist im Verbindungssteg 20 des Haltebügels 14 gelagert. Hierzu kann eine Lagerhülse 34 dienen.

Als Motorantrieb 30 ist bevorzugt ein elektrischer Motor vorgesehen. Stattdessen kann auch ein mechanischer Antrieb, wie insbesondere ein Federwerk, Verwendung finden. Der Motorantrieb 30 ist mit dem Haltebügel 14 verbunden. Vorliegend ist er teilweise im Verbindungssteg 20 des Haltebügels 14 untergebracht. Der Motorantrieb 30 dient dazu, nach Betätigung eines Auslösers 36, z.B. elektrischen Schalters, Tasters oder eines mechanischen Riegels, eine Schwenkbewegung des Ultraschall-Übertragers 8 gegenüber dem Haltebügel 14 um einen Drehwinkel von insgesamt größer/gleich 60° durchzuführen. Der Drehwinkel kann insbesondere zwischen 60° und 90° liegen. Auch eine volle Drehung von 360° ist möglich. In Fig. 2 ist nur der halbe Drehwinkel, der demnach im Bereich zwischen 30° und 45° liegt, durch die Positionen I und II verdeutlicht. Gestartet wird in der Regel in Position I. Der Auslöser 36 dient gleichzeitig auch dazu, daß Echobildaufnahmen nach dem Start der Drehbewegung in verschiedenen Drehwinkelpositionen zwischen 0° und 60° bzw. 90°, d.h. in verschiedenen Schnittebenen, gemacht und elektronisch bis zum Abruf zwecks Auswertung gespeichert werden. Ingangsetzung der Schwenkbewegung und Beginn der Bildaufnahmetätigkeit sind also synchronisiert. Echosichtbild-Aufzeichnungsgeräte sind heutzutage weitgehend standardmäßig mit Bildspeichern ausgerüstet.

Der Auslöser 36 für die Schwenkbewegung und die Bildaufzeichnung - oder nur für eines von beiden - kann prinzipiell ein Fußschalter sein. Bevorzugt ist er aber - wie dargestellt - an der Halterung, also am Haltebügel 14, speziell am Schenkel 16, angeordnet. Er kann somit leicht von der den Schenkel 16 umfassenden Hand des Operateurs bedient werden, sobald eine Aufnahme der Schnittebenen im Bereich des vorgegebenen Drehwinkelbereichs (z.B. von 60° oder 90°) gewünscht ist. Ein gleichartiger Auslöser (nicht gezeigt) kann auch am anderen Schenkel 18 für denselben Zweck angeordnet sein.

Aus Fig. 1 ist ersichtlich, daß die Applikationsfläche 11 des Ultraschall-Übertragers 8 etwas über die Enden der Schenkel 16, 18 des Haltebügels 14 übersteht oder hinausragt. Auf diese Weise ist eine leichte Handhabbarkeit gewährleistet. Denn im Betrieb dreht sich die Applikationsfläche 11 auf der Haut des Patienten 4.

Es soll noch einmal betont werden, daß der Haltebügel 14 frei handhabbar ist. Er ist also z.B. nicht an einem Stativ befestigt. Zwecks Aufsuchens des ersten charakteristischen Punkts P1 kann der Bedienende alle möglichen Bewegungen durchführen, z.B. Verschiebungen der Applikatorfläche 11 parallel zur x,y-Ebene oder Kippbewegungen. Dabei hat er die Schenkel 16, 18 fest in seinen Händen. Ist der Punkt P1 auf dem Bildschirm sichtbar und insbesondere in die Bildmitte gerückt worden, wird der Bedienende den Auslöser 36 betätigen, so daß der Ultraschall-Übertrager 8 eine Schwenkbewegung um den vorgegebenen Drehwinkel Drehwinkel (z.B. 60° oder 90°) durchführt. Während dieser Schwenkbewegung bleibt der Haltebügel 14 ortsfest; es werden verschiedene (z.B. um gleich große (kleine) Winkel auseinanderliegende) Schnittebenen x, z aufgenommen, d.h. die Bildsignale registriert. Aus dem medizinischen Sachverhalt ergibt sich, daß in einer dieser Schnittebenen alle drei Punkte P1, P2, P3 gleichzeitig sichtbar sein müssen. Dies ist die gesuchte Schnittebene. Sie wird zur weiteren Analyse ausgewählt. Nach dem Auslesen kann das betreffende Schnittbild (z.B. bezügl. der Relation und des Abstands der Punkte P1, P2, P3 zueinander) ausgewertet werden. Es gibt somit Aufschluß über Anomalien und Erkrankungen des Hüftgelenks.

In den Figuren 3 und 4 ist eine weitere Ausführungsform eines Ultraschall-Applikators 2 dargestellt. Bei dieser Ausführungsform ist die Halterung speziell als ein ring- oder rahmenförmiger Grundkörper 40 ausgebildet. Im vorliegenden Fall handelt es sich speziell um ein zylindrisches Rohrstück. Auf der einen Stirnseite ist eine starre Membran 42, z.B. aus einem Kunststoff mit akustischen Parametern, die denen des zu untersuchenden Gewebes angepaßt sind (Dichte, Schallgeschwindigkeit), befestigt. Bevorzugt ist die Dicke dieser Membran 42 gleich einem ungeradzahligen Mehrfachen der halben Wellenlänge in diesem Medium (n = 1, 3, 5,). Die Außenseite dieser starren Membran 42 bildet hier die Applikationsfläche 11. An der anderen Stirnseite des Grundkörpers 40 befindet sich eine Verbindungsplatte 44, die dort durch (nicht näher bezeichnete) Schrauben befestigt ist. Im Zentrum dieser Verbindungsplatte 44, und zwar im Innenraum des topfförmigen Gebildes 40, 42, ist durch (nicht näher bezeichnete) Befestigungsmittel der Motorantrieb 30 angeordnet. Dieser ist auch hier wieder in der Lage, den über eine Welle 32 gekoppelten Ultraschall-Übertrager 8 bei Betätigung eines Auslösers 36 (in Fig. 4 gezeigt) von einer Position I in eine Position II zu überführen. Aus Fig. 4 geht hervor, daß diese Schwenkdrehung 90° betragen kann. Statt einer Verbindungsplatte 44 kann auch ein Steg, z.B. in Form eines Metallstreifens, vorgesehen sein.

Auch der in den Figuren 3 und 4 gezeigte Ultraschall-Applikator 2 ist vom Bedienenden frei beweglich. Hierzu dienen zwei bügel-oder henkelförmige Handgriffe 46 und 48, die - in einer Ebene -auf der Außenwand des Grundkörpers 40 symmetrisch befestigt sind. An diesen Handgriffen 46, 48 kann der Arzt den Applikator 2 festhalten. Dies gilt sowohl beim Aufsuchen des ersten interessierenden Punktes P1 als auch bei der Aufnahme von Schnittebenen nach Betätigung des Auslösers 36. Bei der Relativbewegung zwischen dem Ultraschall-Übertrager 8 und dem Grundkörper 40 gleitet die an den Übertragerelementen 10 gelegene Endfläche des Ultraschall-Übertragers 8 über die Innenfläche der Membran 42. Um auch hier eine gute Ankopplung zu gewährleisten, ist zwischen den beiden genannten Flächen ein Ankoppelgel vorgesehen. Entsprechend ist auch die Applikationsfläche 11 am Patienten 4 mit einem Ankoppelgel versehen.

Es soll noch angemerkt werden, daß bei der Ausführungsform nach den Figuren 3 und 4 die Verbindungsplatte 44 entfallen kann, wenn der Motorantrieb 30 an der Innenwand des Grundkörpers 40 befestigt ist. In diesem Fall erhält man einen nach oben hin offenen topfförmigen Applikator 2.

Der Vorteil der in den Figuren 3 und 4 gezeigten Ausführungsform besteht darin, daß keine bewegten Teile auf den Patienten 4 einwirken. Man hat es also mit einer "ruhigen Ankopplung" zu tun.

Dies gilt auch für die Ausführungsform nach Fig. 5. Auch hier ist wieder ein ring-förmiger Grundkörper 40 vorgesehen. An dessen Außenseite sind wiederum zwei Handgriffe 46, 48 befestigt, beispielsweise durch Verschweißen. Bei dieser Ausführungsform ist aber endseitig eine nunmehr flexible Membran 50 vorgesehen. Diese kann beispielsweise aus Gummi bestehen. Im Innenraum des Grundkörpers 40 befindet sich ein Verbindungssteg oder -streifen 52, der zur Durchführung der Welle 32 des außen befestigten Motorantriebs 30 dient. Der von dem Grundkörper 40, von dem Verbindungssteg 52, insbesondere einer Verbindungsplatte, und von der Ankoppelmembran 50 gebildete Innenraum ist mit einem Ankoppelmedium 54, beispielsweise einer Ultraschall gut leitenden Flüssigkeit wie Öl, gefüllt. Die Funktion des Ultraschall-Applikators 2 nach Fig. 5 ist dieselbe wie die in den vorangehenden Figuren 1 bis 4.

Der Vorteil der in Fig. 5 gezeigten Ausführung besteht darin, daß sich die flexible Membran 50 leicht an die Kontur des zu untersuchenden Patienten 4 anschmiegt. Der Bedienende benötigt für die Untersuchung und das Aufsetzen nur eine geringe Anpreßkraft, was insbesondere bei der Untersuchung von Säuglingen von Bedeutung ist.

**Patentansprüche**

1. An ein Echosichtbild-Aufzeichnungsgerät anschließbarer Ultraschall-Applikator (2), insbesondere für die Hüftdiagnose an einem Patienten (4), mit einem Ultraschall-Übertrager (8) zur zeilenweisen Ultraschall-Abtastung, der in einer Halterung (14; 40) gehaltert und durch einen Motorantrieb (30) um eine in der Abtastebene (x, z) gelegene Drehachse (26) drehbar ist, **dadurch gekennzeichnet,** daß die Halterung (14; 40) frei bewegbar und mit zwei in einer Ebene liegenden Handgriffen (16, 18; 46, 48) versehen ist, daß der Ultraschall-Übertrager (8) zwischen den Handgriffen (16, 18; 46, 48) angeordnet und dabei um die Drehachse (26), die parallel zur Ebene der Handgriffe (16, 18; 46, 48) liegt, schwenkbar ist, und daß der Motorantrieb (30) zum Schwenken des Ultraschall-Übertragers (8) um die Drehachse (26) relativ zur Halterung (14; 40) vorgesehen ist (Fig. 1 bis 5).

2. Ultraschall-Applikator nach Anspruch 1, **dadurch gekennzeichnet,** daß die Halterung als U-förmiger, frei bewegbarer Haltebügel (14) ausgebildet ist, daß die Schenkel (16, 18) des Haltebügels (14) als die Handgriffe ausgebildet sind, und daß der Ultraschall-Übertrager (8) zwischen den Schenkeln (16, 18) des Haltebügels (14) angeordnet und dabei um die Drehachse (26), die parallel zu den Schenkeln (16, 18) liegt, schwenkbar ist (Fig. 1 und 2).

3. Ultraschall-Applikator nach Anspruch 1, **dadurch gekennzeichnet,** daß die Halterung einen starren ringförmigen Grundkörper (40) aufweist, an dessen Außenseite die Handgriffe (46, 48) angebracht sind (Fig. 3 bis 5).

4. Ultraschall-Applikator nach einem der Ansprüche 1 bis 3, **dadurch gekennzeichnet,** daß an zumindest einem Handgriff (16; 46, 48) ein Auslöser (36) für die Schwenkbewegung und/oder die Bildaufzeichnung angeordnet ist (Fig. 1 bis 5).

5. Ultraschall-Applikator nach Anspruch 4, **da-**

durch gekennzeichnet, daß der Motorantrieb (30) so ausgebildet ist, daß bei Betätigung des Auslösers (36) eine Schwenkbewegung um einen Drehwinkel von insgesamt größer oder gleich 60° erfolgt, wobei gleichzeitig Echobildaufnahmen in verschiedenen Schnittebenen durchgeführt werden (Fig. 1 bis 5).

6. Ultraschall-Applikator nach einem der Ansprüche 1 bis 5, **dadurch gekennzeichnet,** daß der Motorantrieb (30) ein elektrischer Motor oder ein Federwerk ist (Fig. 1 bis 5).

7. Ultraschall-Applikator nach einem der Ansprüche 1 bis 6, **dadurch gekennzeichnet,** daß der Motorantrieb (30) in der Halterung (14; 40) untergebracht ist (Fig. 1 bis 5).

8. Ultraschall-Applikator nach einem der Ansprüche 1 bis 7, **dadurch gekennzeichnet,** daß als Ultraschall-Übertrager (8) ein lineares Array vorgesehen ist (Fig. 1 bis 5).

9. Ultraschall-Applikator nach einem der Ansprüche 1 bis 8, **dadurch gekennzeichnet,** daß zum Schwenken um die Drehachse (26) eine Welle (32) den Motorantrieb (30) mit dem Ultraschall-Übertrager (8) verbindet, und daß die Welle (32) in einem Verbindungssteg (20; 52) der Halterung (14; 40) gelagert ist (Fig. 1, 2 und 5).

10. Ultraschall-Applikator nach einem der Ansprüche 2 bis 9, ausgenommen Anspruch 3, **dadurch gekennzeichnet,** daß die Schenkel (16, 18) des Haltebügels (14) zwecks Ausbildung als Handgriffe mit Rundungen (22, 24) versehen sind (Fig. 2).

11. Ultraschall-Applikator nach einem der Ansprüche 1 bis 10, ausgenommen Anspruch 3, **dadurch gekennzeichnet,** daß die Applikationsfläche (11) des Ultraschall-Übertragers (8) über die Enden der Schenkel (16, 18) des Haltebügels (14) übersteht (Fig. 1).

12. Ultraschall-Applikator nach einem der Ansprüche 3 bis 9, **dadurch gekennzeichnet,** daß die Handgriffe (46, 48) bügelförmig ausgebildet sind (Fig. 3 bis 5).

13. Ultraschall-Applikator nach einem der Ansprüche 3 bis 9 oder 12, **dadurch gekennzeichnet,** daß der Grundkörper (40) endseitig eine starre Membran (42) aufweist, an der der Ultraschall-Übertrager (8) anliegt (Fig. 3 und 4).

14. Ultraschall-Applikator nach einem der Ansprüche 3 bis 9 oder 12, **dadurch gekennzeichnet,** daß der Grundkörper (40) endseitig eine flexible Membran (50) aufweist und mit einer Ankoppelsubstanz (54) zwischen dem Ultraschall-Übertrager (8) und der Membran (54) gefüllt ist (Fig. 5).

15. Ultraschall-Applikator nach einem der Ansprüche 3 bis 9 oder 12, **dadurch gekennzeichnet,** daß im Grundkörper (40) eine Verbindungsplatte (44; 52) angeordnet ist, an der der Motorantrieb (30) befestigt ist (Fig. 3 und 5).

16. Verfahren zum Betrieb des Ultraschall-Applikators (2) nach einem der Ansprüche 1 bis 15, **dadurch gekennzeichnet,**
    a) daß die Applikationsfläche (11) auf das zu untersuchende Objekt (4) aufgesetzt wird, wobei der Ultraschall-Übertrager (8) gegenüber der Halterung (14; 40) arretiert ist,
    b) daß der Applikator (2) auf dem Objekt (4) verschoben wird, bis ein interessierender Punkt (P1) auf dem Sichtbild erscheint,
    c) daß das Schwenken des Ultraschall-Übertragers (8) um die Drehachse (26) ausgelöst wird,
    d) daß beim Schwenken die Handgriffe (16, 18; 46, 48) festgehalten sowie gleichzeitig Schnittbild-Aufnahmen gemacht und aufgezeichnet werden, und
    e) daß die Schnittbild-Aufnahmen nacheinander aus dem Bildspeicher abgerufen werden.

**Claims**

1. Ultrasonic applicator (2) which can be connected to an echo-image recording device, in particular for the hip diagnosis of a patient (4), having an ultrasonic transmitter (8) for linear ultrasonic scanning, which is held in a holder (14; 40) and can be rotated by means of a motor drive (30) about an axis of rotation (26) placed in the scanning plane (x, z), characterized in that the holder (14; 40) can be moved freely and is provided with two handles (16, 18; 46, 48) lying in a plane, in that the ultrasonic transmitter (8) is arranged between the handles (16, 18; 46, 48) and in this respect can be pivoted about the axis of rotation (26), which lies parallel to the plane of the handles (16, 18; 46, 48), and in that the motor drive (30) for the pivoting of the ultrasonic transmitter (8) about the axis of rotation (26) is provided relative to the holder (14; 40) (Figures 1 to 5).

2. Ultrasonic applicator according to claim 1, characterized in that the holder is constructed as a U-shaped, freely moveable support bracket (14), in that the limbs (16, 18) of the support bracket (14) are constructed as the handles, and in that the ultrasonic transmitter (8) is arranged between the limbs (16, 18) of the support bracket (14) and in this respect can be pivoted about the axis of rotation (26) which lies parallel to the limbs (16, 18) (Figures 1 and 2).

3. Ultrasonic applicator according to claim 1, characterized in that the holder has a rigid, annular base (40), on the outer side of which are fitted the handles (46, 48) (Figures 3 to 5).

4. Ultrasonic applicator according to one of claims 1 to 3, characterized in that a trigger device (36) for the pivot movement and/or the image recording is arranged on at least one handle (16; 46, 48) (Figures 1 to 5).

5. Ultrasonic applicator according to claim 4, characterized in that the motor drive (30) is constructed in such a way that with the actuation of the trigger device (36) a pivot movement about an angle of rotation of in total greater than or the same as 60° takes place, whereby at the same time echo-image recordings are carried out in various sectional planes (Figures 1 to 5).

6. Ultrasonic applicator according to one of claims 1 to 5, characterized in that the motor drive (30) is an electric motor or a spring mechanism (Figures 1 to 5).

7. Ultrasonic applicator according to one of claims 1 to 6, characterized in that the motor drive (30) is accommodated in the holder (14; 40) (Figures 1 to 5).

8. Ultrasonic applicator according to one of claims 1 to 7, characterized in that as ultrasonic transmitter (8) a linear array is provided (Figures 1 to 5).

9. Ultrasonic applicator according to one of claims 1 to 8, characterized in that for the pivoting about the axis of rotation (26) a shaft (32) connects the motor drive (30) to the ultrasonic transmitter (8), and in that the shaft (32) is mounted in a connecting bar (20; 52) of the holder (14; 40) (Figures 1, 2 and 5).

10. Ultrasonic applicator according to one of claims 2 to 9, excluding claim 3, characterized in that the limbs (16, 18) of the support bracket (14) are provided with rounded areas (22, 24) for the purpose of construction as handles (Figure 2).

11. Ultrasonic applicator according to one of claims 1 to 10, excluding claim 3, characterized in that the application face (11) of the ultrasonic transmitter (8) projects over the ends of the limbs (16, 18) of the support bracket (14) (Figure 1).

12. Ultrasonic applicator according to one of claims 3 to 9, characterized in that the handles (46, 48) are constructed in the shape of support brackets (Figures 3 to 5).

13. Ultrasonic applicator according to one of claims 3 to 9 or 12, characterized in that the base (40) has on the end side a rigid diaphragm (42), against which the ultrasonic transmitter (8) rests (Figures 3 and 4).

14. Ultrasonic applicator according to one of claims 3 to 9 or 12, characterized in that the base (40) has on the end side a flexible diaphragm (50) and between the ultrasonic transmitter (8) and the diaphragm (54) [sic] is filled with a coupling substance (54) (Figure 5).

15. Ultrasonic applicator according to one of claims 3 to 9 or 12, characterized in that in the base (40) a connection plate (44; 52) is arranged, to which the motor drive (30) is fastened (Figures 3 and 5).

16. Method for operating the ultrasonic applicator (2) according to one of claims 1 to 15, characterized in that
a) the application face (11) is mounted on to the object (4) to be examined, whereby the ultrasonic transmitter (8) is secured opposite the holder (14; 40),
b) the applicator (2) is displaced on the object (4) until an interesting point (P1) appears on the image,
c) the pivoting of the ultrasonic transmitter (8) about the axis of rotation (26) is initiated,
d) during the pivoting, the handles (16, 18; 46, 48) are secured and at the same time sectional images are taken and recorded, and
e) the sectional images are successively retrieved from the image memory.

## Revendications

1. Applicateur à ultrasons (2) pouvant être raccordé à un appareil d'enregistrement d'images d'échos, notamment pour le diagnostic de la hanche d'un patient (4), comportant un transducteur à ultrasons (8) pour réaliser une exploration ultrasonique par balayage ligne par ligne et qui est fixé dans un support (14;40) et peut être entraîné en rotation par un dispositif d'entraînement à moteur (30) autour d'un axe de rotation (26) situé dans le plan de balayage (x,z), caractérisé par le fait que le dispositif de fixation (14;40) est librement mobile et est pourvu de deux poignées (16,18;46,48) situées dans un plan, que le transducteur à ultrasons (8) est disposé entre les poignées (16,18; 46,48) et peut pivoter autour de l'axe de rotation (26), qui est parallèle au plan des poignées (16,18;46,48), et que le dispositif d'entraînement à moteur (30) est prévu pour faire pivoter le transducteur à ultrasons (8) autour de l'axe de rotation (26) par rapport au dispositif de fixation (14;40) (figures 1 à 5).

2. Applicateur à ultrasons suivant la revendication 1, caractérisé par le fait que le dispositif de fixation est réalisé sous la forme d'un étrier de retenue en forme de U (14) librement mobile, que les branches (16,18) de l'étrier de retenue (14) sont réalisées sous la forme de poignées, et que le transducteur à ultrasons (8) est disposé entre les branches (16,18) de l'étrier de fixation (14) et peut pivoter autour de l'axe de rotation (26), qui est parallèle aux branches (16,18) (figures 1 et 2).

3. Applicateur à ultrasons suivant la revendication 1, caractérisé par le fait que le dispositif de fixation possède un corps de base annulaire rigide (40), sur la face extérieure duquel sont montées les poignées (46,48) (figures 3 à 5).

4. Applicateur à ultrasons suivant l'une des revendications 1 à 3, caractérisé par le fait qu'un déclencheur (36) pour le mouvement de pivotement et/ou l'enregistrement d'images est monté sur au moins une poignée (16;46,48) (figures 1 à 5).

5. Applicateur à ultrasons suivant la revendication 4, caractérisé par le fait que le dispositif d'entraînement à moteur (3) et agencé de telle sorte que, lors de l'actionnement du déclencheur (36), on obtient un mouvement de pivotement sur un angle de rotation globalement supérieur ou égal à 60°, les enregistrements d'images d'échos étant exécutés simultanément dans différents plans de coupe (figures 1 à 5).

6. Applicateur à ultrasons suivant l'une des revendications 1 à 5, caractérisé par le fait que le dispositif d'entraînement à moteur (3) est un moteur électrique ou un dispositif d'entraînement à ressort (figures 1 à 5).

7. Applicateur à ultrasons suivant l'une des revendications 1 à 6, caractérisé par le fait que le dispositif d'entraînement à moteur (3) est logé dans le dispositif de fixation (14;40) (figures 1 à 5).

8. Applicateur à ultrasons suivant l'une des revendications 1 à 7, caractérisé par le fait qu'il est prévu, comme transducteur à ultrasons (8), un réseau linéaire (figures 1 à 5).

9. Applicateur à ultrasons suivant l'une des revendications 1 à 8, caractérisé par le fait que pour le pivotement autour de l'axe de rotation (26), un arbre (32) relie le dispositif d'entraînement à moteur (30) au transducteur à ultrasons (8) et que l'arbre (32) est monté dans une barrette de jonction (20;52) du dispositif de fixation (14;40) (figures 1, 2 et 5).

10. Applicateur à ultrasons suivant l'une des revendications 2 à 9, exceptée la revendication 3, caractérisé par le fait que, pour la réalisation des poignées, les branches (16,18) de l'étrier de retenue (14) comportent des arrondis (22,24) (figure 2).

11. Applicateur à ultrasons suivant l'une des revendications 1 à 10, à l'exception de la revendication 3, caractérisé par le fait que la surface d'application (11) du transducteur à ultrasons (8) fait saillie au-delà des extrémités des branches (16,18) de l'étrier de retenue (14) (figure 1).

12. Applicateur à ultrasons suivant l'une des revendications 3 à 9, caractérisé par le fait que les poignées (46,48) sont réalisées sous la forme d'étriers (figures 3 à 5).

13. Applicateur à ultrasons suivant l'une des revendications 3 à 9 ou 12, caractérisé par le fait que le corps de base (40) possède, à son extrémité, une membrane rigide (42), contre laquelle est appliqué le transducteur à ultrasons (8) figures 3 et 4.

14. Applicateur à ultrasons suivant l'une des revendications 3 à 9 ou 12, caractérisé par le fait que le corps de base (40) comporte, à son extrémité, une membrane flexible (50) et est rempli par une substance de couplage (54) disposée entre le transducteur à ultrasons (8) et la membrane (54) (figure 5).

15. Applicateur à ultrasons suivant l'une des revendications 3 à 9 ou 12, caractérisé par le fait que dans le corps de base (40) est disposée une plaque de liaison (44;52), à laquelle est fixé le dispositif d'entraînement à moteur (30) (figures 3 et 5).

16. Procédé pour faire fonctionner l'applicateur à ultrasons (2) suivant l'une des revendications 1 à 15, caractérisé par le fait

a) qu'on dispose la surface d'application (11) sur l'objet à examiner (4), le transducteur à ultrasons (8) étant bloqué par rapport au dispositif de fixation (14;40),

b) qu'on déplace l'applicateur (2) sur l'objet (4) jusqu'à ce qu'un point intéressant (P1) apparaisse sur l'image,

c) qu'on déclenche le pivotement du transducteur à ultrasons (8) autour de l'axe de rotation (26),

d) que lors du pivotement, on maintient fermement les poignées (16,18,46,48) et on prend et on enregistre simultanément des enregistrements d'images de coupe, et

e) qu'on appelle les enregistrements d'images de coupe successivement à partir de la mémoire d'images.

EP 0 295 521 B1

FIG 1

FIG 2

FIG 3

FIG 4

FIG 5